Europäisches Patentamt

(19) **European Patent Office**

Office européen des brevets

(11) Publication number: **0 255 487**
**A2**

(12) # EUROPEAN PATENT APPLICATION

(21) Application number: **87810432.2**

(51) Int. Cl.⁴: **A 63 B 21/00**

(22) Date of filing: **29.07.87**

(30) Priority: **01.08.86 CH 3095/86**

(43) Date of publication of application:
**03.02.88 Bulletin 88/05**

(84) Designated Contracting States:
**AT BE DE ES FR GB GR IT LU NL SE**

(71) Applicant: **Petralli, Carlo**
**Via agli Orti 20**
**CH-6962 Viganello (CH)**

(72) Inventor: **Petralli, Carlo**
**Via agli Orti, 20**
**CH-6962 Viganello (CH)**

(74) Representative: **Pozzoli, Giuliano**
**e/o ENGIMPEX S.A. Engineering Consultants & Industrial**
**Management P.O. Box 18**
**CH-6902 Lugano-Paradiso (CH)**

(54) **Physiotherapeutic electronic device to recover the limbs and to restore the skeleton and muscular functions.**

(57) The device includes various groups (M1, M1a, M2, M3, M4, M5, M6, M7, P8, P9, P10, P11, EO1, SM1, BO1, CP1), modular, compatible and suitable to be integrated in an unique structure, each for a definite function; drived by permanent electric motors or by hydraulic rams or by pneumatic cylinders with proportional velves, assisted by a system software.

The tools or accessories, each specialized to rehabilitate the function of a muscle or limb, first of all allow the dynamometric survey of the function of the patient to be treated.

Measuring sensors send the data to a processing unit providing first the compilation of the patient card and the programming of the relevant therapy.

Then from the measured and programmed data a system software provides to assign to the tools, through the motors, the positions, rotations, extensions and to calibrate the effort proportionally and gradually. Unlike the devices with passive principle, where the patient works on the tool, with the induced physical and psychic difficulties, arising during the practice of the exercise, the suject device acts itself on the patient, bringing him gradually to the prompt recovery of the function to be restored (fig. 1).

EP 0 255 487 A2

## Description

Physiotherapeutic electronic device to recover the limbs and to restore the skeleton and muscular functions

The present invention refers to a modular physiotherapeutic device to functionally recover the limbs and to restore the skeleton and muscular functions injured by sickness or by accident, including in a single structure all the anatomical dynamometric tools and accessories and controlling and measuring devices, each related to a predetermined function.

The tools are drived by permanent electric micromotors or oleopneumatic cylinders controlled in extension and intensity by a system software interfacing with a programme collecting the data of the patient, compiling his electronic card and preparing the session to be executed, after the collection by the same tools, by means of the sensors connected to them, of the general status of the patient, establishing the therapy card. The device subject of the invention is based on the integration with devices and tools, partially new, being also part of the present invention, partially already known, of servoassisted electric or hydraulic micromotors, capable to drive the tool itself in extension and with the desidered intensity under the control of a system software interfacing to a programme with the card of the patient as input, collected and compiled by the device itself.

It's well-known from the technique that "passive type" physiotherapeutic devices do exist; in these the effort calibration happens before the exercise by means of the adjustement or the variation of the elastic characteristic of the elastic means (springs) or by means of brakes or clutches. Besides it they are usually monofunctional, namely employed to the recovery of a single muscular or skeleton function or to the recovery of a single organ (hand, arm, leg) or at the best they group a number of similar functions of muscular groups in a localized zone of the patient body. Instead other devices limit themselves only to the dynamometric measurement of the muscular and functional capability. More precisely the present invention refers, on the contrary, to a physiotherapeutic device for the functional recovery of the limbs and the restoration of the original mobility of the skeleton and muscular functions, injured by sickness or by accident, including a number of dynamometric anatomical tools, each for the restoration and recovery of a definite motory and muscular function, characterized by the fact that these tools are connected to a system of control of the effort intensity drived by motors, servoassisted, programmed and controlled by a system software, in order to allow the various muscles and/or limbs to achieve those isometric, isostatic, ergodynamic and ergonomic movements, necessary to their original functional recovery, after the collection and measurement, by means of the tools themselves, of the conditions of the functions of the patient, then establishing the card of the physiotherapeutic session(s).

It is well suitable for use in establishments of the physiotherapeutic area, special gymnasia, establishments for disabled people, schools, sporting clubs for its peculiarly good results, particularly based on its level integration and refinement.

It's a matter then of an active recovery device, because the tools work on the patient after the surveying of the general data of the patient functions and of the function to be restored and the operator must only send back to the system software the already prepared card. Moreover it's a matter of a decomposable modular device, capable of integration with other functions or usable just partially for the desidered functions. It can be used simultaneously by many user too.

The dynamic electronic device subject of the invention uses a rational system, drived by servoassisted permanent motors under the control of a microprocessor. At present the device can carry out 100 implemented functions. Further options are possible in the ambit of this invention.

It has been especially studied for the rehabilitation of limbs with extremities articulated on the human body, for the controlled therapy, rotational, linear, with pressure, with expansion, with traction; moreover it is possible to combine it with devices of the last generation in order to perform the cardiac checks and to measure the blood pressure, the number of pulsations, the muscular strength, the angle of movement, the weight, the height and further parameters of the skeleton and muscular apparatus.

The device subject of the invention is structured as a modular system called "Bau-Kasten"; lower basement by welded solid metal, surmounted by three special columns with incorporated magnetic motors, assuring the working positioning in elevation; upper table with special framework by oxidized aluminum, carrying all the working units, with special handles.

In a special version a pneumatic-electronic system has been integrated, assuring with the aid of the compressor the air under pressure necessary for the opearation of the pneumatics.

The device subject of the invention has been studied and structured with twelve main working stations and more precisely:

1) M1 rotary unit, two directions, limb hands
2) M2 rotary unit, two directions, shoulders arms
3) M3 rotary unit, two directions, legs feet
4) M4 linear unit, two directions, shoulders arms hands
5) M5 linear unit, two directions, arms legs
6) M6 linear unit, two directions, positioning
7) M7 rotary unit, two directions, head cervical hands feet
8) P8 linear unit, traction extension, two positions
9) P9 linear unit, extension pressure, two positions

10) P10 linear unit, traction

11) P11 linear unit, synchronized alternate rythm

12) M1a/M4/P9 linear unit, spiral rotary alternate advancement

The principle of operation is as follows: initial check-up, passive diagnosis, measurement of the limb movement and muscle force are recorded according to predetermined card and programme, according to the data taken from the active or passive patient; among them just those relevant to the system of working programme are updated and selected, in order to establish proportionally the effort to be applied and the number of cycles; or by means of the microprocessor with acoustic signals and video displaying of the whole subroutine which signals, conducts and controls the whole therapeutic session, with the indication of the start and the end of the exercise, number of times, applid effort, service unit and so on.

By a video mean, monitor or display, it is possible to analyse the working phases in real time with instantaneous technique; finally the acoustic signal disappears and then the operational unit stops; it is also possible to print the data of the sessions executed on each unit, prearranging a printing mean (printer).

The integrated multifunctional physiotherapeutic device is particularly suitable for:

Active - Passive

M1 Abduction - adduction - flexion

M1a/M4 Supinating - pronating - abduction - adduction - extension - flexion - eccentric - concentric

M1/P9 Extension - flexion - adduction - traction - pressure - rotation

M2/M5 Flexion - compression - extension - flexion - adduction - ratation - abduction

M5 Sartorius - extension - flexion

M7 Flexion - rotation

M3 Flexion - extension - ergometric - traction - pressure - rotation - eccentric - concentric

M6 Ergometric

P4/P8/P10 Traction

Neck flexion

Neck extension

Trunk flexion

Trunk extension

Trunk rotation

Pelvis elevation

Thigh flexion

Thigh extension

Thigh abduction

Arm extension

Arm flexion

Arm abduction in the horizontal plane

Arm adduction in the horizontal plane

Arm external rotation

Arm internal rotation

Forearm flexion

Forearm extension

Forearm supinating

Forearm pronating

Hand flexion with abduction

Hand flexion with adduction

Hand extension with abduction

Hand extension with adduction

Flexion of the first phalanges of the last four fingers of the hand

Flexion of the second and third phalanges of the last four fingers of the hand

Abduction of the hand fingers

Adduction of the hand fingers

Thumb abduction

Thumb adduction

Opposition of the thumb and the little finger

Thumb phalanges flexion

The physiotherapeutic device subject of the invention is a valid help in the rehabilitation of the people suffering a casualty or a sickness, due to its proportional characteristics, namely stress progressive control according to the programme. It provides the assistance with precise and automatic rhythm, in terms of number of revolutions, angles and linear displacements, leading to an improved well-being of the concerned limb, following a treatment logic; on the other end the patient can at any time exercise himself in opposition to the work phase and doing in this way he engages a physical muscular resistance of contraction of the limb, feeling a real improvement.

The device can also be programmed to work under conditions of total resistance and in opposition to the movements of the limbs; allowing in such a way an intensive physical exercise, producing well-being and prompt recovery.

The subject device can guarantee the assistance to at least four people simultaneously with diversification of therapy or of programmed exercise.

Moreover it is equipped with an interface for connection with large centres of data processing, by hospitals, specialized establishments and so on; in this way it assures the management of the treatment programme, providing data, survey of patients, progress of the work to the attending personnel and costs, lists, invoicing and so on to the administrative services.

With a closed circuit system the remote assistance can be realized.

Conclusively it is a matter of a very versatile, complete, well equipped, reliable device, unique in its kind, allowing the patient to know with stated precision his physical state in real time, before, during and after the exercise, contributing in this way to a personal improvement and creating confidence and reliance in the used mean; these are important psychological conditions for a prompt recovery.

It follows a detailed description of a preferred, although not limiting, form of execution of the subject of the invention with reference to the attached tables, however without depriving anything to the generality of the invention itself.

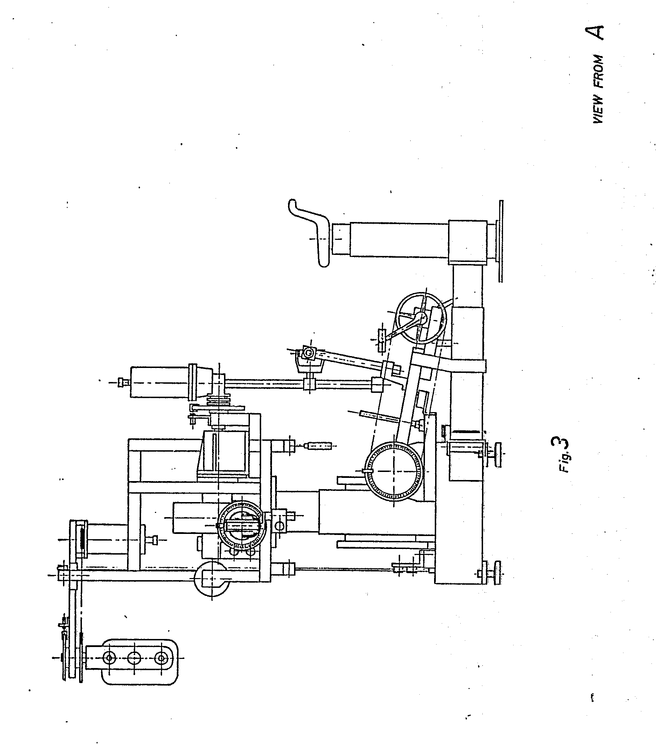

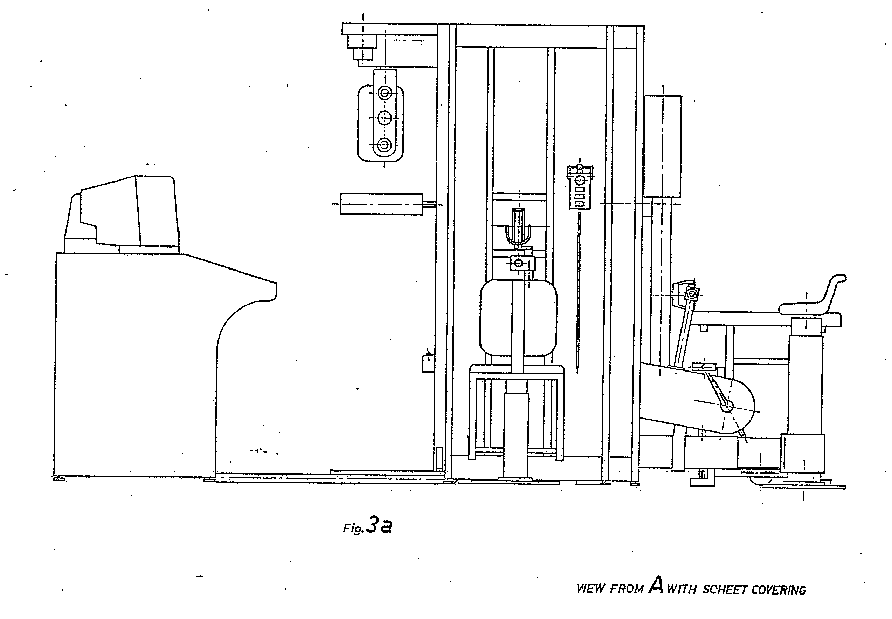

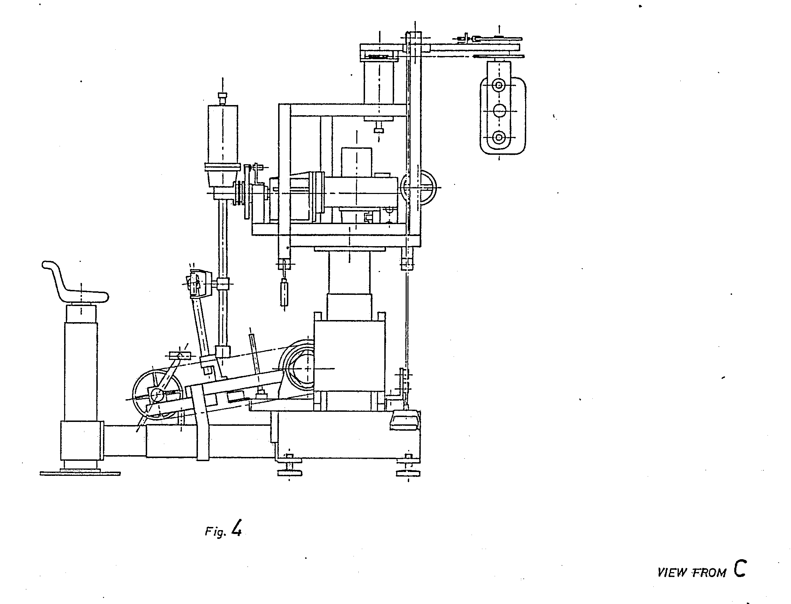

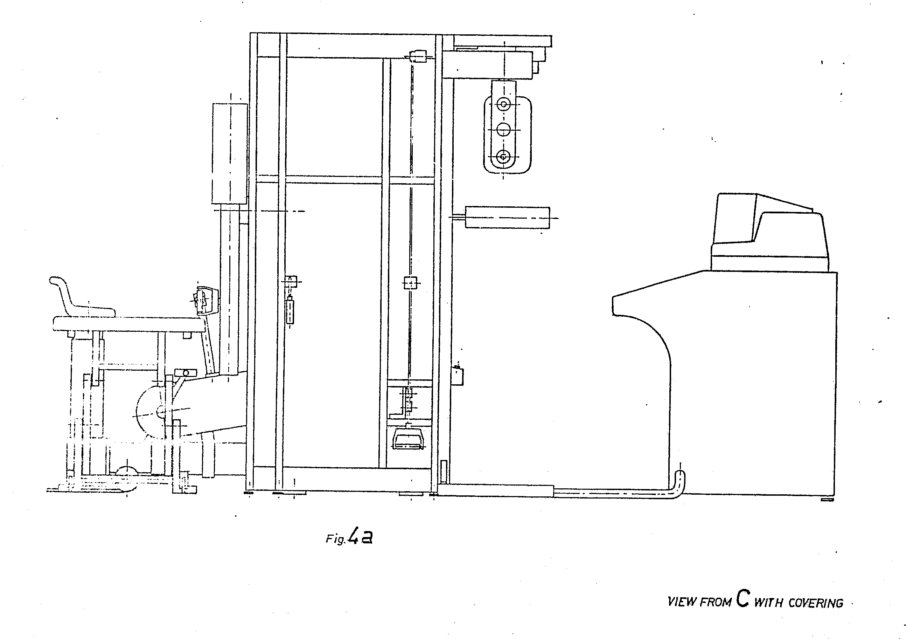

- The figures 1, 1a, 2, 2a, 3, 3a, 4 and 4a show respectively front view, top view and side views of the device subject of the invention.

- The figures 5a and 5b show the source of electric and/or hydraulic-pneumatic power, respectively in front and side view.

- The figures 6a and 6b show respectively the data processing, surveying and control unit, in front and side view.

- The figures 7a and 7b show the group lying table, respectively in front and side view.

- The figures 8a and 8b show the bi-directional rotary unit, limb and hands, respectively in front and side view.

- The figures 9a and 9b show the bi-directional rotary unit, shoulder and arms, and the linear unit, arms and legs, respectively.

- The figures 10a and 10b show, respectively in front and side view, the bi-directional rotary unit, legs and feet, with pedals and flywheel.

- The figures 11a and 11b show in front and side view respectively the bi-directional rotary unit, head neck hands feet.

- The figures 12a and 12b show respectively in front and side view the two-positions linear unit for traction and extension.

- The figures 13a and 13b show respectively in front and side view the linear unit with rotary alternate advancement.

## 1. Foreword

For the physiotherapy it is interesting to have available a device drived and controlled by a computerized system, capable to simulate as exactly as possible a body therapy.

The muscular and articular rehabilitation of a person, which, let's say, has suffered a casualty, requires the performance of reiterated motory exercises, described with the highest precision, which can be a part of a long-term rehabilitation process.

The restoration of the corporal functions of a person is still the duty of a physiotherapist.

It is not yet available on the market a device drived by a computerized system which can contribute to a rehabilitation of this kind according to the indications of a physiotherapist and which can control the rehabilitation progress from the beginning till the end. It is for this reason that the device subject of the invention has been studied.

Thanks to this device the different movements are executed with the aid of electric motors or pneumatic or hydraulic cylinders.

The motor driving and the therapy control occur by means of an effective computer (even a Personal Computer). The link man-device occurs by means of the keyboard, possibly of a Personal Computer. A software package fit for the physiotherapy allows the physiotherapist to understand as widely as possible, together with a simplified control of the device.

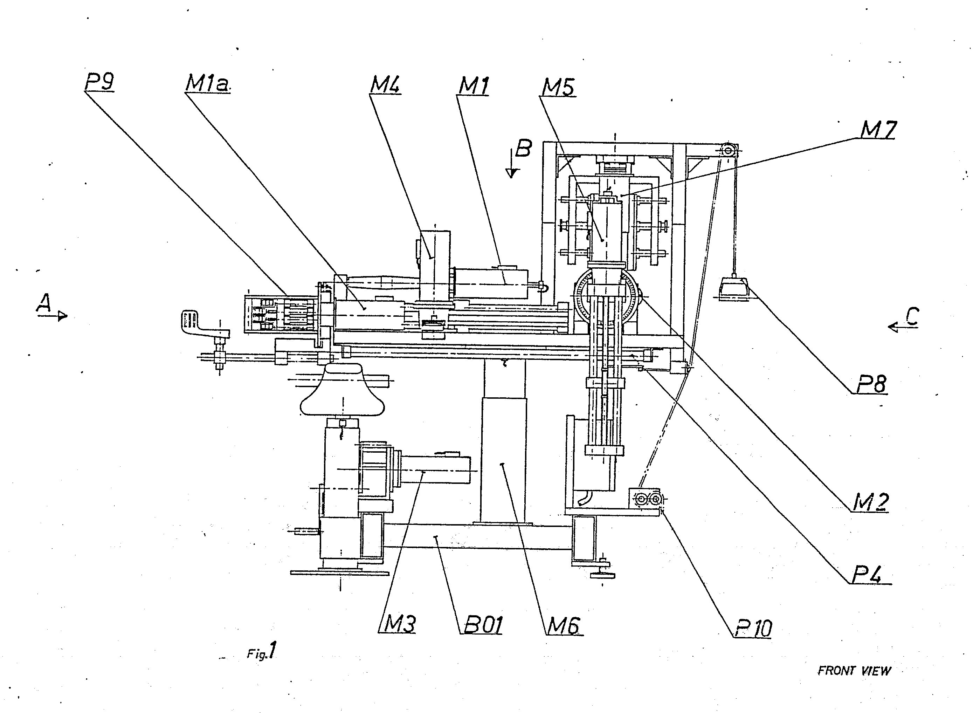

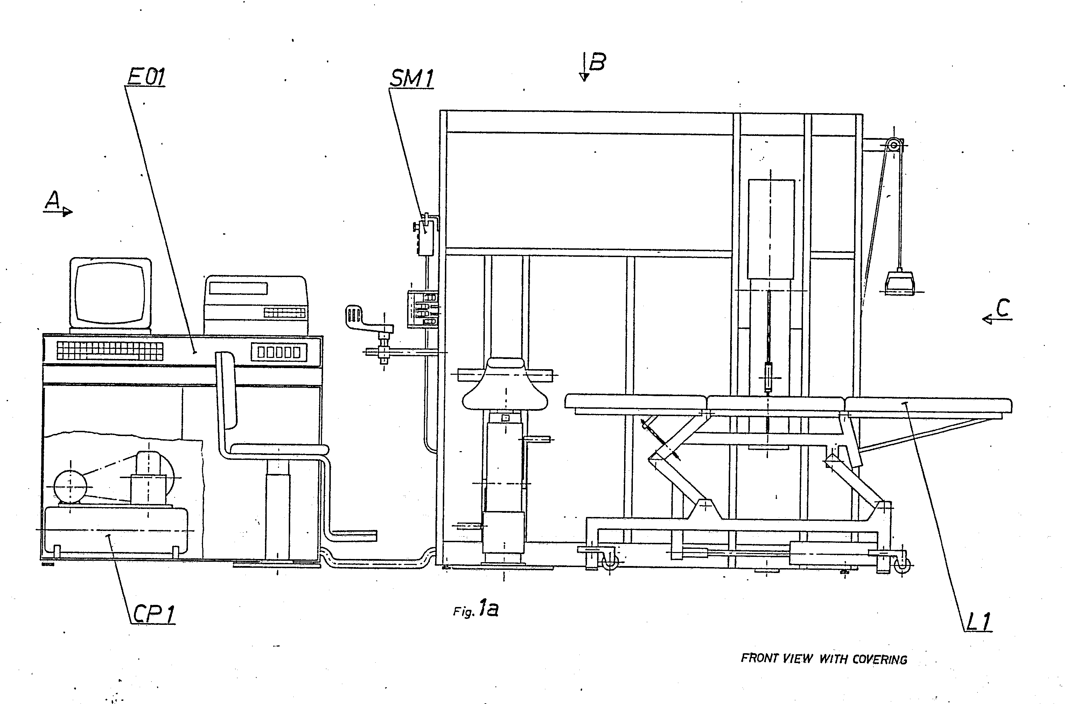

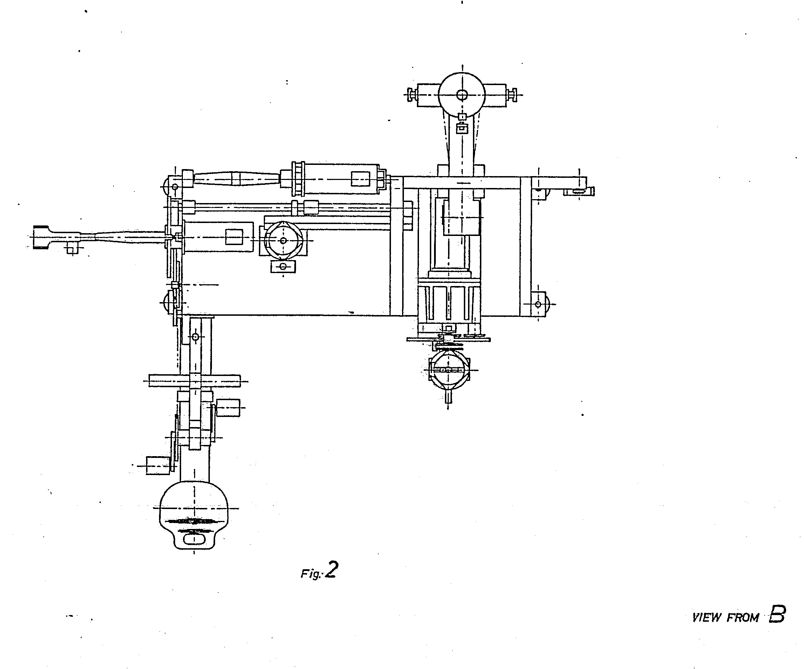

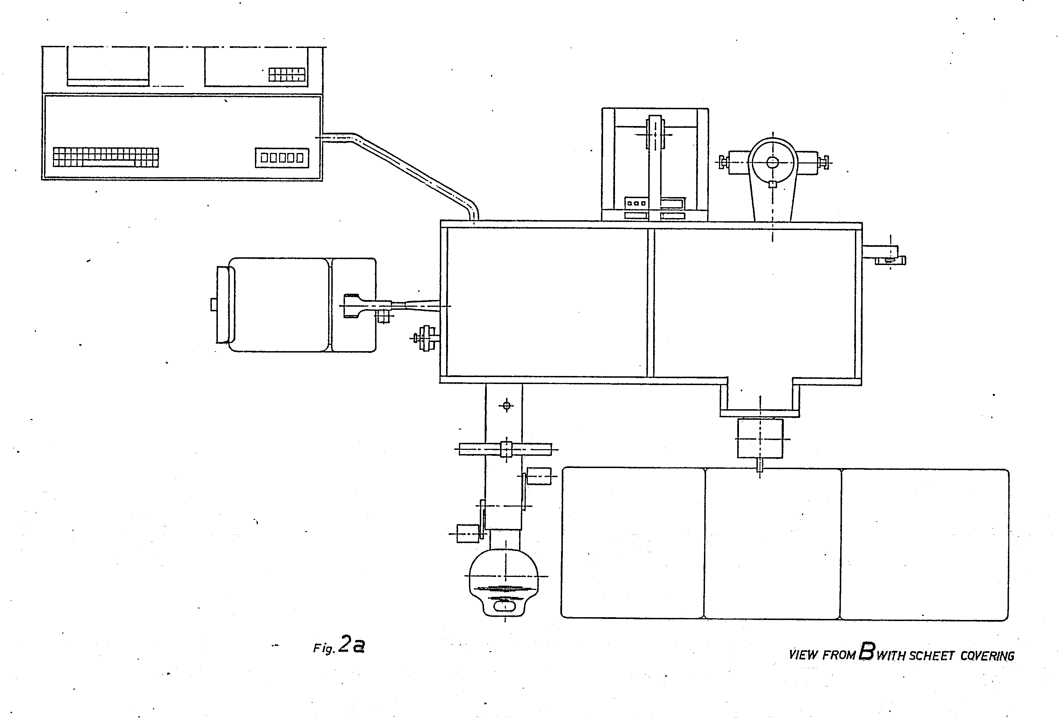

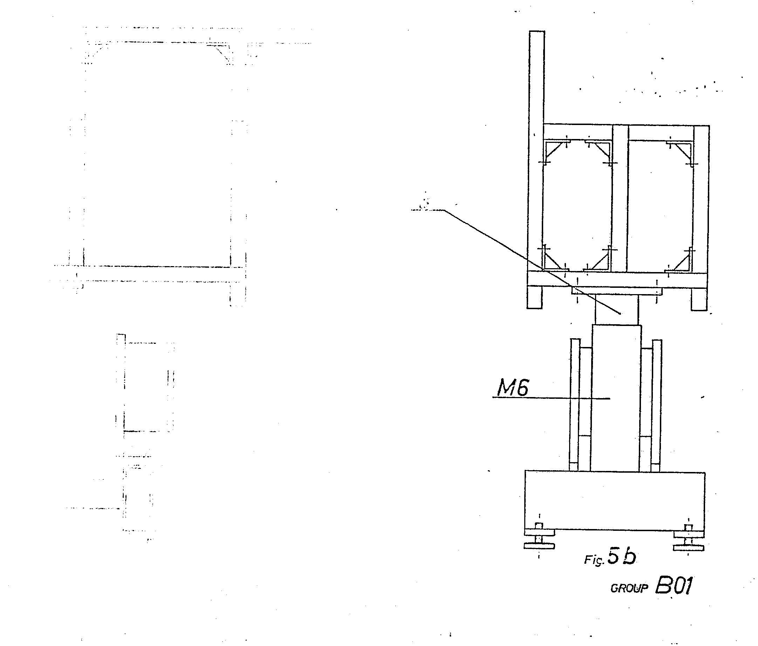

The device subject of the invention includes a number of tools and groups M1, M1a, M2, M3, M4, M5, M6, M7, P8, P9, P10, P11, EO1, SM1, BO1, CP1 (fig. 1), each for a function, or linkable together for a defined function. The group CP1 (figures 5a and 5b) includes the power source of the device formed by the feeding of the electric micromotors, by the hydraulic or pneumatic feeding and by the feeding circuit of servoassistance 1, 2. It is mounted on the lower base of the carrying structure or frame of the device 3.

The group BO1 (figures 5a and 5b) includes, in addition to the frame 3, the group M6 for the positioning of the patient on the tools according to his size.

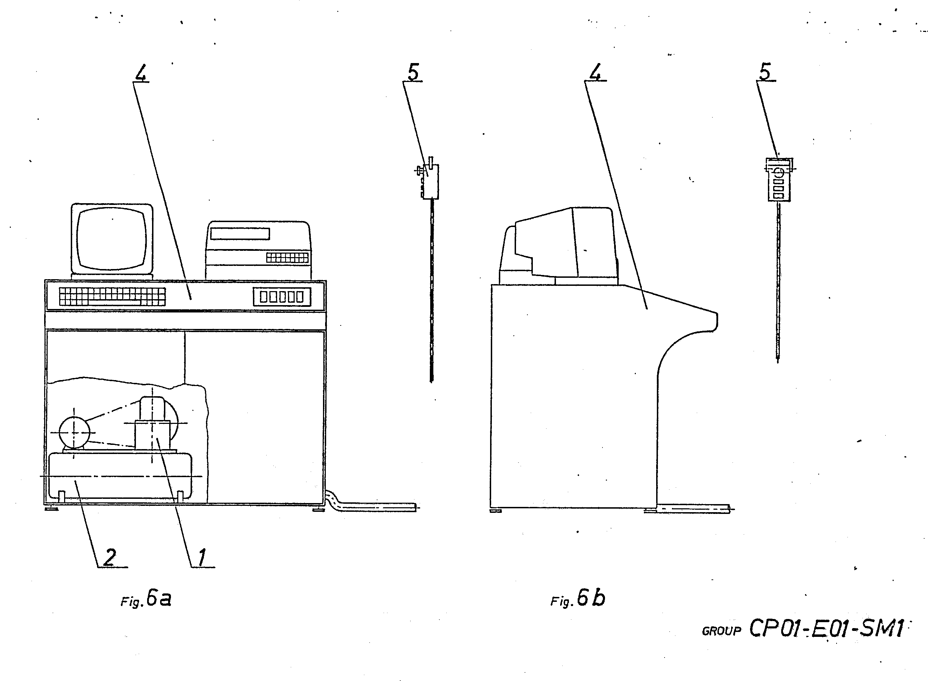

The group EO1 and the group SM1 linked together form the surveying, processing, input information and data management centre 4. A video screen or display allows the operator and the patient to visualize the data

4

and the card and to follow the treatement during the therapy (figures 6a and 6b).

A printing unit can be connected to the central computer. Moreover it is also foreseen a link with the central elaboration units of large data processing centres, like sickness funds, hospitals, polyclinics and so on.

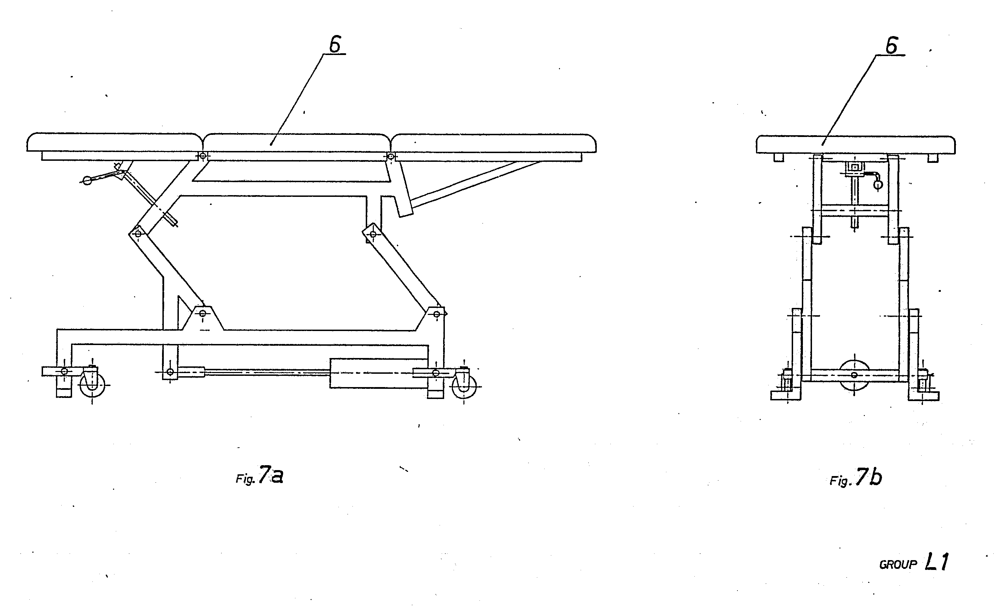

The group L1 (figures 7a and 7b) is formed by a lying table or bed 6 on rolling frame.

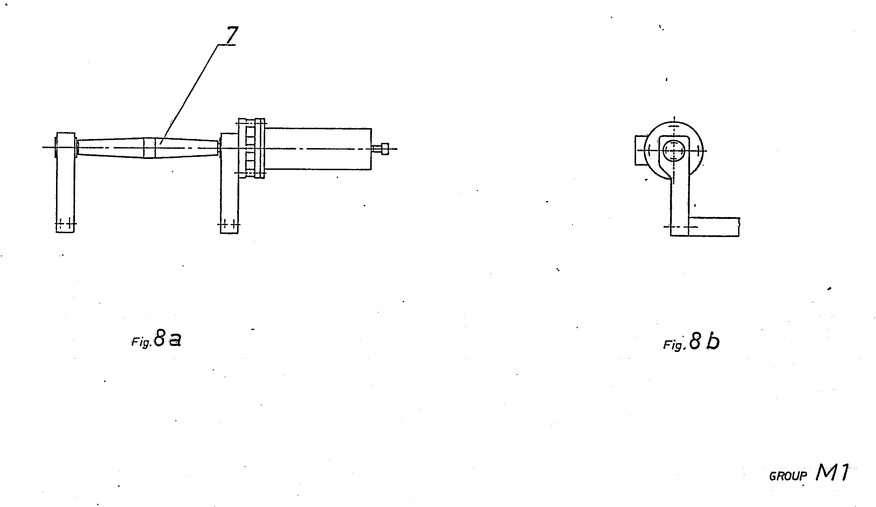

The group M1 (figures 8a and 8b) forms the bi-directional rotary unit for limb and hand 7, allowing the hand and at the same time the forearm to reach with gradual effort left-hand and right-hand angular rotations.

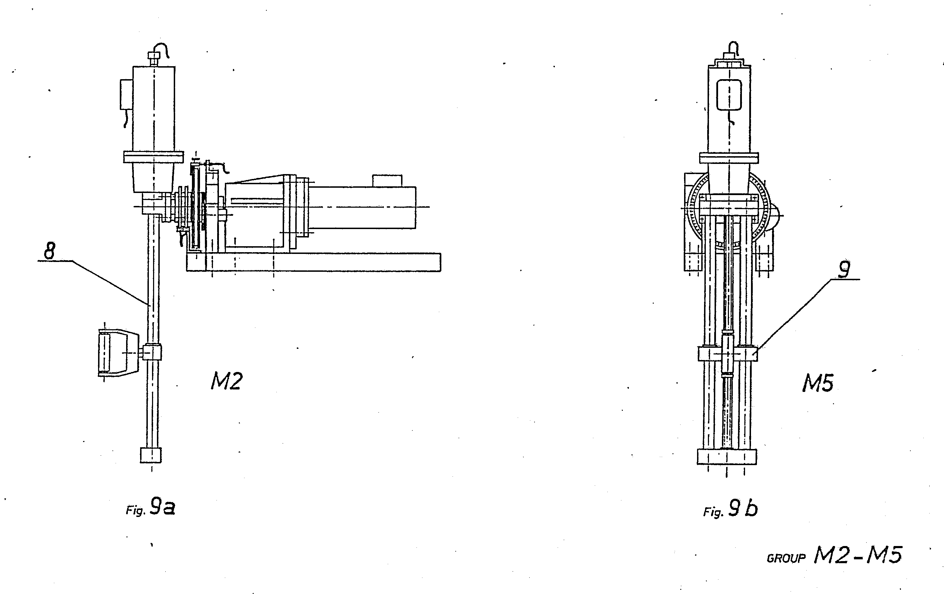

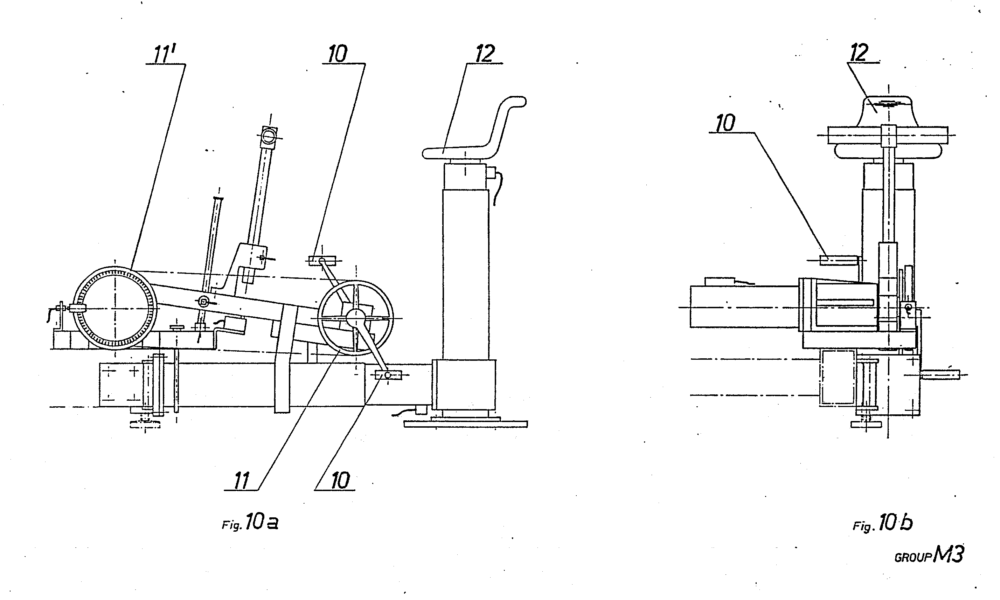

The group M2 is formed by a bi-directional rotary unit for shoulder and arms 8, whereas the group M5 is formed by a linear unit for arms and legs (figures 9a and 9b). The group M3 forms the bi-directional rotary unit for legs and feet (figures 10a and 10b) including pedals 19 on disk 11 mechanically connected to a disk linked to the permanent micromotor which drives and calibrates the effort. A chair adjustable in height and on the two flat frames (3 positions) completes the group.

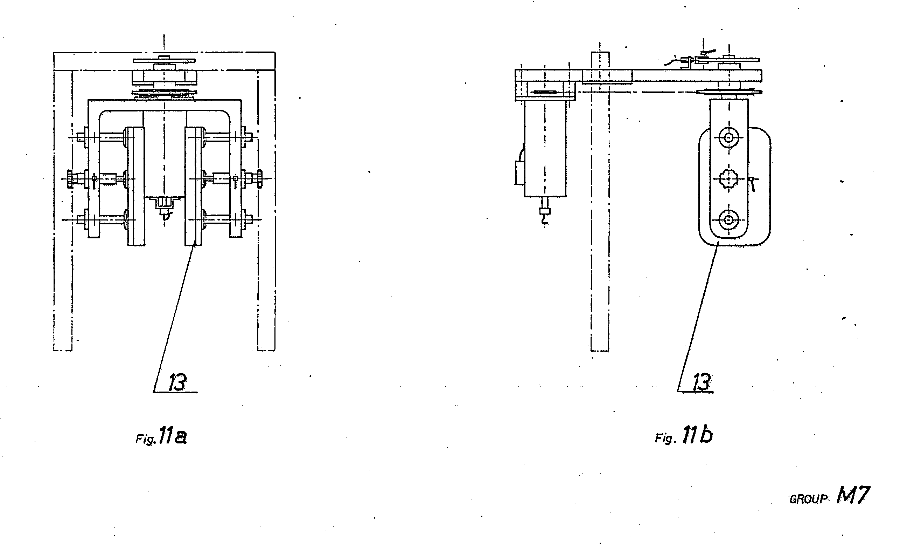

The group M7 (figures 11a and 11b) allows the therapy of head, neck, hands and feet by means of the bi-directional rotary unit connected to the dedicated tool.

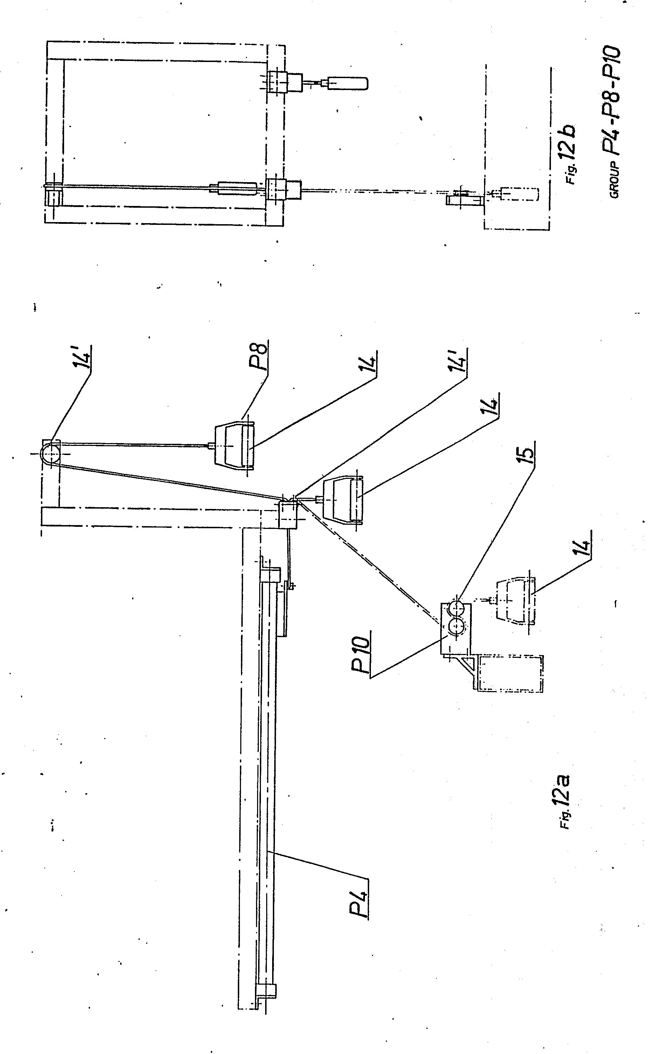

The groups P8 - P10 form two-positions linear units, traction and extension, for hands, arms, feet and legs by means of handles 14 connected and drived by pulleis 14' with power take-off from a micromotor 15 (figures 12a and 12b). Replacing the two handles with a collar tool, this unit can be used for the function head, neck, nape.

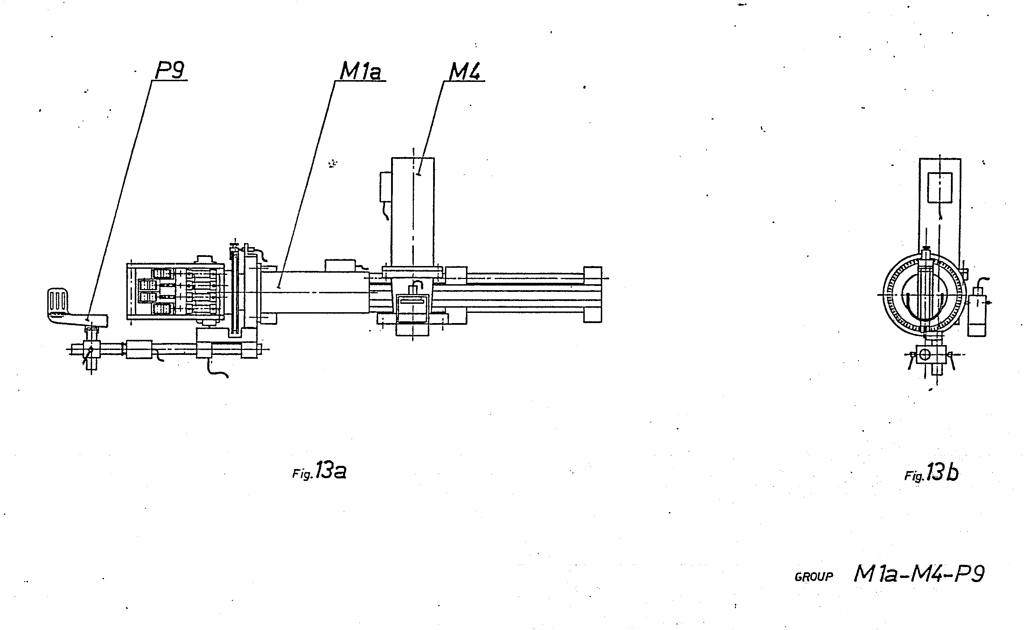

The groups M1a - M4 - P9 combined together form a linear unit with alternate rotary advancement (figures 13a and 13b).

## 2. Hardware functions

### 2.1. Main electric functions

The motors M1, M2, M3, M4 and M7 are permanent magnet and direct current motors. This kind of motor allows to control with the highest accuracy both the torque and the speed.

With the aid of an additional electronic device it is also possible to obtain the positioning of the rotation angle.

### 2.1.1. Block diagram

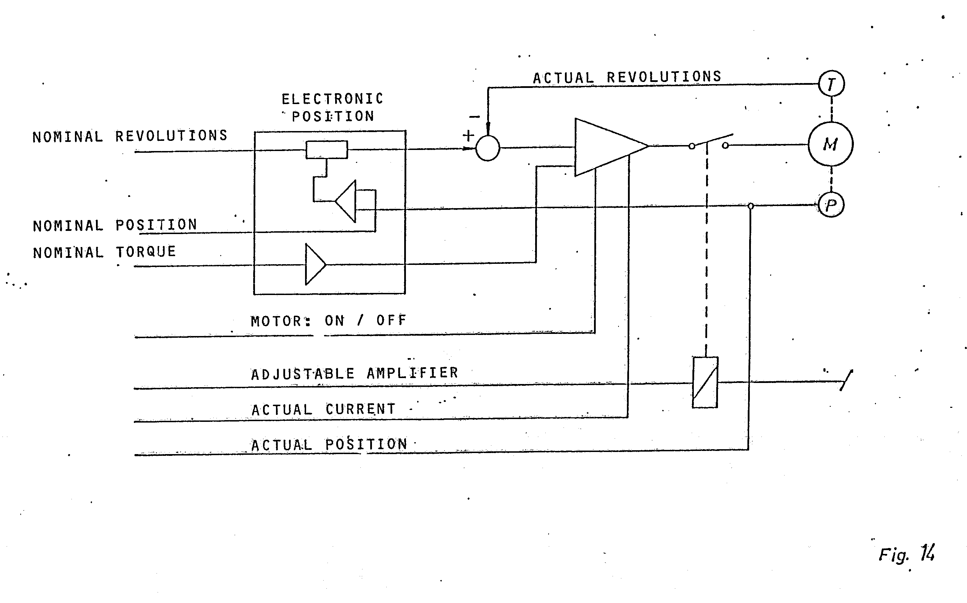

See fig. 14.

### 2.1.2. Control of the motors

The block diagram shows the mode of operation of the control of the motors:

a) Electronics for positioning: it compares the analogue nominal and actual values and adapts the output signals to the amplifier of the motors.

b) Amplifier of the motors: it amplifies the input analogue signals and starts the motor with the output signal.

c) Actual value generation: T = tacheometer, generates an output tension proportional to the rotation angle; the analogue control channels are closed with the recycling of the actual values.

### 2.1.3. Nominal values

The following nominal values are introduced on the computer:

a) number of revolutions (0-100%)

b) position (0-360°)

c) torque

These signals are defined as analogue control signals.

### 2.1.4. Analogue measuring signals

The two measuring signals "actual current value" and "actual position" are directly introduced in the computer to allow the performance of the power and positioning calculation.

### 2.1.5. Digital control signals

A motor can be started or stopped by means of two digital control signals.

### 2.2. Auxiliary electric functions

The motors M6.1, M6.2 and M6.3 provide the auxiliary electric functions. These three alternate current motors are equivalent one to the other. They serve to adapt the tool to the size of the patient.

### 2.2.1. Block diagram

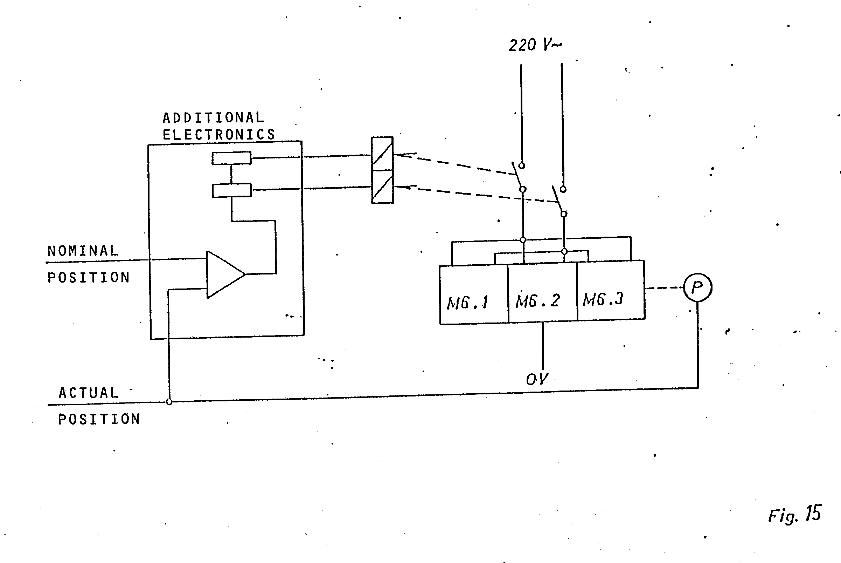

See fig. 15.

### 2.2.2. Control of the motors

This simplified control is only related to the position in elevation of the device with a three-positions regulator. The motors turn at the maximum sped forward or backward and develop automatically the torque necessary to the minimum lifting of the device. The position is supplied by the computer.

2.3. Pneumatic functions
The pneumatic functions P8 and P9 are executed by means of pneumatic cylinders. The pneumatic cylinders control occurs by means of electro-pneumatic components.

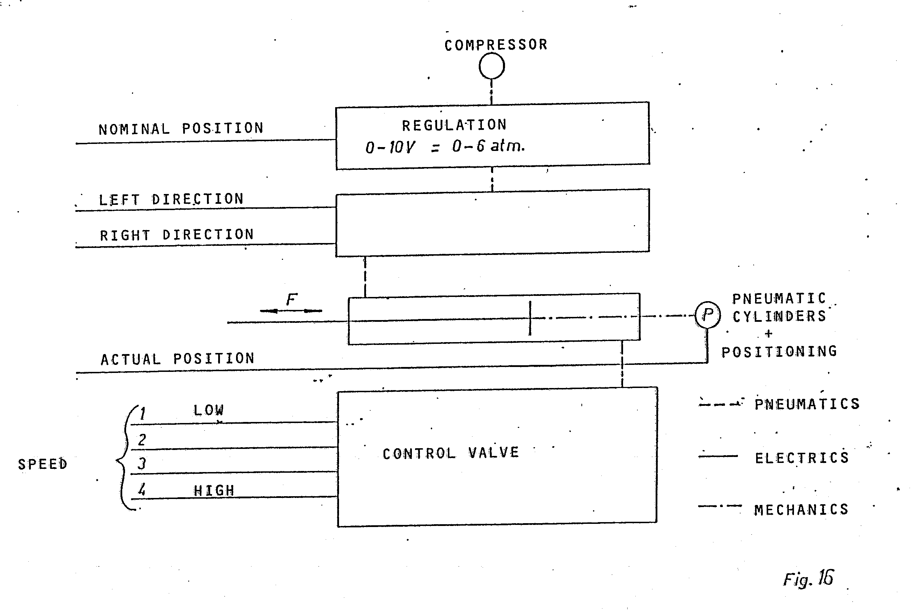

2.3.1 Block diagram
See fig. 16.

2.3.2 Control of the cylinders
a) Converter: the pneumatic pressure of operation is defined by the electro-pneumatic converter by means of the analogue tension "give pressure".
b) Directional block: the direction of the movement of the cylinders can be determined by means of two valves.
c) Pneumatic cylinders: in the pneumatic cylinder it is induced a force F proportional to the pressure of operation.
c) Throttle valves: by means of these valves four cylinders speeds can be choosen.

2.3.3 Control signals
a) Analogue control signals: - give pressure
b) Digital control signals:
- left direction
- right direction
- 4 control signals for 4 cylinders speeds

2.3.4 Analogue measuring signals
Cylinder stroke: this analogue value is read by the computer and processed by the aid of the software.

3. Security measures
The security of the device and of the people working on it is of extreme importance; for this reason we put particular emphasis on this factor, creating and including in the device suitable active and passive means.

3.1. Security measures for the user
Keys and switches desactivating immediately the device:
- EMERGENCY STOP
- STOP
- various MANUAL KEYS
Foreseen electric limitations:
- maximum number of revolutions of the motors
- maximum torque of the motors

3.2. Security measures
All the metallic parts of the device are connected to the earth.
Security automatisms protecting every single part of the device from the current overload.
Galvanic separation between mains and device.

4. Computer and interface

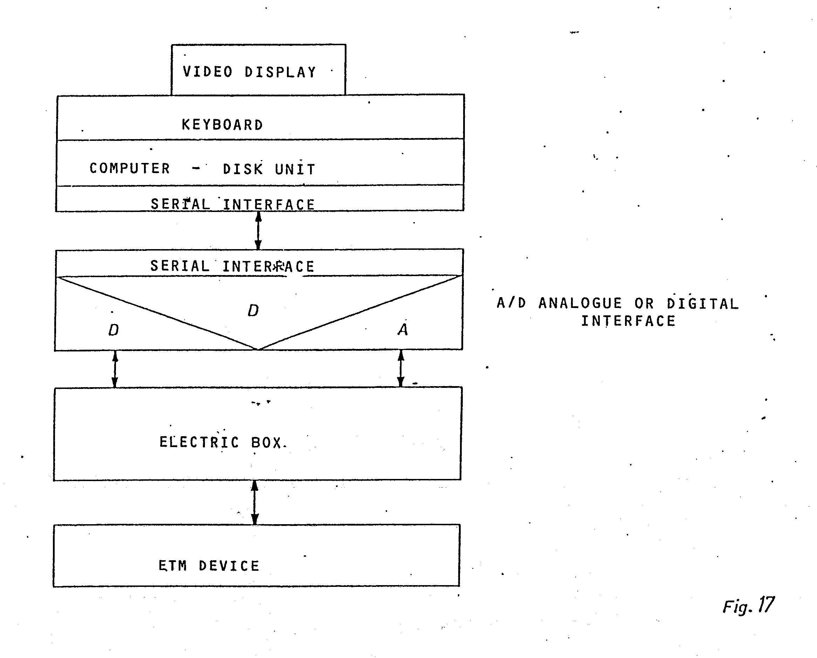

4.1. Block diagram
See fig. 17.

4.2. Mode of operation
The data flow from the interface to the computer and vice versa occurs by means of a standard serial interface (RS-232).
The orders are given to the device by means of a keyboard.
The computer processes, adapts and transmits them.
The interface puts the data in analogue form or generates the digital control orders. The analogue or digital signals arrive in the distribution box and control the motors and the pneumatic cylinders. The measuring signals arrive through the distribution box to the A/D interface, where they become digital and are transferred to the computer.
The management of the device, the data transmission, their evaluation and their management occur by means of a programme.

5. Software
The software controls all the transport units of the device and manages the data in a mass memory (flexible disk).

**0 255 487**

The programme is written in BASIC language and stored on a disk under the name "ETM-100".

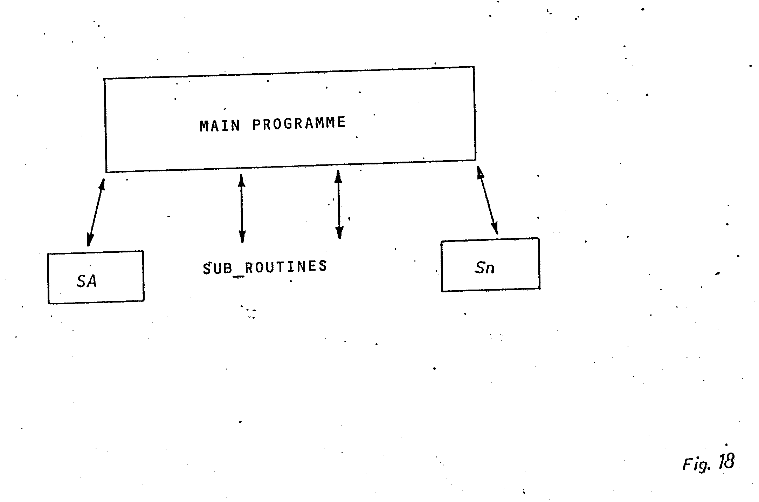

5.1. Programme structure
See fig. 18.
The programme includes a main programme and a certain number of sub-routines.
The main programme calls the sub-routines and let them work.

5.2. Man-device link
The programme is structured with the "menu" technique.
That means that the screen shows in plain form which data the user must input.
The user can follow the work progress even without any knowledge of the programme language. The measures are shown on the screen (Macintosh-type).

5.3. Input and output parameters
The following electric parameters can be inserted by the user:
- output position
- left starting position
- right starting position
- rotation speed
- return speed
- torque
- number of repetitions
The following pneumatic parameters can be inserted by the user:
- pneumatic pressure
- pneumatic speed (4 stages)
Just one order can be inserted. The transport choice occurs by means of the list of operational options provided by the programme.
The following measures are shown on the screen:
- power or weight acting on the motor (power measurement through the measurement of the motor current)
- actual position of the cylinders or of the motors

5.4. Example of programme development

| Order to the computer | Description |
|---|---|
| LOAD ETM-100 (CR) (name of the programme) | The programme is loaded by the disk of working memory (disk in A drive) |
| RUN | The programme is executed |
| Display on the screen | Input through keyboard |
| Pneumatic or electric (PNEUMATIC/ELECTRIC) | Choice between motors and cylinders, for instance ELECTRIC |
| If ELECTRIC: | |
| Position = ? | See limits for numerical |

7

| | |
|---|---|
| (0-360)  (0-100%) | values input |
| Number of revolutions<br>= ?  (0-100%) | Ditto |
| Torque = ? (0-100%) | Ditto |
| Pneumatic pressure = ?<br>(0-100%) | Ditto |
| Motor = ? (1,2,3,4,5,6) | Ditto |
| Positioning executed | |
| Other functions (YN) | Choice of the letter Y or<br>N (yes or no) |
| Programme continuation<br>(YN) | Choice of the letter Y or<br>N (yes or no) . |
| Start of the list: | |
| 1. Motors movement with<br>muscular force | Choice of the development<br>1,2 or 3 |
| 2. The motors move by<br>themselves | For instance 2 |
| 3. Power measurement | |
| Left position = ?<br>(0-360) | Input of the numerical<br>value of the rotation<br>angle in a defined<br>direction, measured from<br>the instantaneous point |

```
                              of exit, for instance 90

Right position = ?           Ditto, for instance 50
(0-360)


Rotation speed = ?           Input of the number of
(0-100%)                     revolutions of the motor,
                             for instance 10


Power = ? (0-100%)           Power to be supplied by
                             the motor, for instance 5


Number of cycles = ?         For instance 20


At this point the motor executes the desidered
movements and announces with an acoustic signal the
end of the cycle.
The operational options can be carried out just
with this procedure.


Note: All the data inputs through the keyboard must
       end with the key "Carriage Return".
```

### 5.5. Future software developments

The sofware will be continually updated according to the requirements of the physiotherapists. The necessary principles have been elaborated in cooperation with physiotherapists.

## Claims

1) Physiotherapeutic electronic device to functionally recover the limbs and to restore the original mobility of the skeleton and muscular functions, injured by sickness or by accident, including a number of anatomical dynamometric tools, each for the restoration and recovery of a definite motory and muscular function, characterized by the fact that these tools are connected to a system which controls the intensity of the effort, drived by motors servoassisted, programmed and controlled by a system software, to allow the various muscles and/or limbs to achieve those concentric, eccentric, isometric isostatic, isokinetic, ergodynamic and ergonomic movements, necessary to their original functional recovery, after the collection and measurement by the same tools of the conditions of the patient functions, establishing the card of the physiotherapeutic session(s).

2) Physiotherapeutic device according to the Claim 1, in which the motors are permanent electric micromotors, in order to calibrate the effort of the patient.

3) Physiotherapeutic device according to the Claim 1, characterized by the fact that the said motors are hydraulic, in order to calibrate, direct and stabilize the effort of the patient.

4) Device according to the Claim 1, characterized by the fact that the above mentioned motors are pneumatic cylinders with proportional valves, in order to calibrate, direct and stabilize the effort of the patient.

5) Physiotherapeutic device according to the Claim 1, 2, 3 or 4, characterized by the fact that it includes a system software, integrated with the device, capable to let the device execute a complete therapy session by means of the movements, rotations, extensions with predetermined instensity, imparted to the micromotors according to a workcard of the patient and to the relevant interface software.

6) Physiotherapeutic device according to the Claim 5, characterized by the inclusion of a central processing interface for the global control of the treatement by hospitals, sickness funds, nursing homes or multifunctional centres with statistics, therapy planning and invoicing.

7) Physiotherapeutic device according to the Claim 1, in which a function is constituted by a bi-directional rotary unit (M1) for limb and hands with cylindrical and/or biconical mandrel.

8) Physiotherapeutic device according to the Claim 1, in which a function is constituted by a bi-directional rotary unit (M2) for shoulders and arms.

9) Physiotherapeutic device according to the Claim 1, in which a function is constituted by a bi-directional rotary unit (M3) for legs and feet.

10) Physiotherapeutic device according to the Claim 1, in which a function is constituted by a linear unit (M5) for arms and legs.

11) Physiotherapeutic device according to the Claim 1, in which a function is constituted by a bi-directional rotary unit for head, neck, hands, feet.

12) Physiotherapeutic device according to the Claim 1, in which a function is constituted by the integration of two linear units for traction/extension with two positions (P8 / P10).

13) Physiotherapeutic device according to the Claim 1, in which a function is constituted by a linear unit with rotary alternate advancement (M1a / M4 / M9).

14) Physiotherapeutic device according to the Claim 1, in which the functions are modular, suitable to be assembled and integrated together.

Fig.1

FRONT VIEW

P9    M1a    M4    M1    M5    M7

B

A

C

P8

M2

P4

M3    B01    M6    P10

0255487

E01

SM1

A

B

CP1

Fig. 1a

L1

C

FRONT VIEW WITH COVERING

0255487

Fig. 2

VIEW FROM B

0255487

VIEW FROM *B* WITH SCHEET COVERING

Fig. 2a

0255487

0255487

VIEW FROM A

Fig.3

0255487

VIEW FROM A WITH SCHEET COVERING

Fig. 3a

Fig. 4

VIEW FROM C

0255487

0255487

VIEW FROM C WITH COVERING

Fig.4a

0255487

Fig. 5b

GROUP B01

M6

4

5

4

5

2

1

Fig. 6a

Fig. 6b

GROUP CP01-E01-SM1

0255487

Fig. 7b

Fig. 7a

GROUP M1

Fig. 8b

Fig. 8a

M2

8

Fig. 9a

M5

9

Fig. 9 b

GROUP M2-M5

0255487

Fig. 10 a

Fig. 10 b

GROUP M3

Fig. *11a*

Fig. *11b*

0255487

Fig. 12 a

P4

P10

P8

14'

14

14'

14

15

14

Fig. 12 b

GROUP P4-P8-P10

0255487

P9    M1a    M4

Fig. *13a*

Fig. *13b*

0255487

GROUP  *M1a-M4-P9*

ACTUAL REVOLUTIONS

ELECTRONIC POSITION

NOMINAL REVOLUTIONS

NOMINAL POSITION

NOMINAL TORQUE

MOTOR: ON / OFF

ADJUSTABLE AMPLIFIER

ACTUAL CURRENT

ACTUAL POSITION

Fig. 14

220 V~

ADDITIONAL
ELECTRONICS

NOMINAL

POSITION

ACTUAL

POSITION

M6.1    M6.2    M6.3

P

OV

0255487

Fig. 15

COMPRESSOR

NOMINAL POSITION

REGULATION
$0-10V = 0-6 atm.$

LEFT DIRECTION

RIGHT DIRECTION

F

PNEUMATIC
CYLINDERS
+
POSITIONING

P

ACTUAL POSITION

SPEED

1 LOW
2
3
4 HIGH

CONTROL VALVE

- - - PNEUMATICS

——— ELECTRICS

—·— MECHANICS

Fig. 16

VIDEO DISPLAY

KEYBOARD

COMPUTER — DISK UNIT

SERIAL INTERFACE

SERIAL INTERFACE

D

D                    A

A/D ANALOGUE OR DIGITAL
INTERFACE

ELECTRIC BOX.

ETM DEVICE

Fig. 17

MAIN PROGRAMME

SA        SUB_ROUTINES        Sn

Fig. 18